# EUROPÄISCHE PATENTSCHRIFT

(11) **EP 2 079 679 B1**
(45) Veröffentlichungstag und Bekanntmachung des Hinweises auf die Patenterteilung: **22.08.2012**
(21) Anmeldenummer: 07818755.6
(22) Anmeldetag: 05.10.2007
(51) Int. Cl.: C07C 231/02, C07C 233/65

(54) **VERFAHREN ZUR HERSTELLUNG TERTIÄRER AMIDE VON ALKYLPHENYLCARBONSÄUREN**
METHOD FOR PRODUCING TERTIARY AMIDES OF ALKYLPHENYL CARBOXYLIC ACIDS
PROCÉDÉ DE PRODUCTION D'AMIDES TERTIAIRES D'ACIDES ALKYLPHÉNYLCARBOXYLIQUES

(30) Priorität: 09.10.2006 DE 102006047620
(43) Veröffentlichungstag der Anmeldung: 22.07.2009
(73) Patentinhaber: Clariant Finance (BVI) Limited, Road Town, Tortola (VG)
(72) Erfinder: KRULL, Matthias, 55296 Harxheim (DE); MORSCHHÄUSER, Roman, 55122 Mainz (DE); LERCH, Alexander, 63571 Gelnhausen (DE); RITTER, Helmut, 42111 Wuppertal (DE); SCHMITZ, Sarah, 45130 Essen (DE)
(74) Vertreter: Mikulecky, Klaus
(86) Internationale Anmeldenummer: PCT/EP2007/008679
(87) Internationale Veröffentlichungsnummer: WO 2008/043494

(56) Entgegenhaltungen:
- NERY, MARCELO S. ET AL: "Niobium pentachloride promoted conversion of carboxylic acids to carboxamides: Synthesis of the 4-aryl-1,2,3,4-tetrahydroisoquinoline alkaloid structures" SYNTHESIS, (2), 272 -276 CODEN: SYNTBF; ISSN: 0039-7881, 2003, XP002465741
- GELENS E ET AL: "An atom efficient and solvent-free synthesis of structurally diverse amides using microwaves" TETRAHEDRON LETTERS, ELSEVIER, AMSTERDAM, NL, Bd. 46, Nr. 21, 23. Mai 2005 (2005-05-23), Seiten 3751-3754, XP004858119 ISSN: 0040-4039 in der Anmeldung erwähnt
- VAZQUEZ-TATO M P: "MICROWAVE-MEDIATED SYNTHESIS OF AMIDES" SYNLETT, THIEME INTERNATIONAL, STUTTGART, DE, Nr. 7, 1993, Seite 506, XP002056049 ISSN: 0936-5214
- WU, XIONGYU ET AL: "Microwave -Enhanced Aminocarbonylations in Water" ORGANIC LETTERS , 7(15), 3327-3329 CODEN: ORLEF7; ISSN: 1523-7060, 2005, XP002465742

## Beschreibung

Tertiäre Amide von Alkylphenylcarbonsäuren sind eine pharmakologisch wie auch technisch sehr interessante Verbindungsklasse. Beispielsweise finden Amide von Alkylbenzoesäuren mit sekundären Alkylaminen als Insektenabwehrmittel (Repellent) Verwendung.

Für die Herstellung von Amiden aromatischer Carbonsäuren wurden verschiedene Methoden entwickelt. Bisher ist man dabei auf kostenintensive und langwierige Herstellverfahren angewiesen, um eine kommerziell interessante Ausbeute zu erzielen. Die bekannten Herstellverfahren erfordern hoch reaktive Carbonsäurederivate wie beispielsweise Säureanhydride, Säurehalogenide wie zum Beispiel Säurechloride, Ester bzw. eine in-situ-Aktivierung durch den Einsatz von Kupplungsreagentien wie beispielsweise N,N'-Dicyclohexylcarbodiimid oder sehr spezielle und damit teure Katalysatoren. Bei diesen Herstellverfahren entstehen zum Teil große Mengen unerwünschter Nebenprodukte wie Alkohole, Säuren und Salze, die vom Produkt abgetrennt und entsorgt werden müssen. Aber auch die in den Produkten verbleibenden Reste dieser Hilfs- und Nebenprodukte können zum Teil sehr unerwünschte Effekte bewirken. So führen beispielsweise Halogenidionen wie auch Säuren zu Korrosion. Kupplungsreagentien sowie die von ihnen gebildeten Nebenprodukte sind zum Teil giftig, sensibilisierend oder carcinogen.

Um die Effizienz von Synthesen zu erhöhen und die Menge der zu entsorgenden Nebenprodukte zu vermindern, werden neue Methoden gesucht, um tertiäre Amide direkt aus Alkylphenylcarbonsäure und sekundärem Amin ohne Einsatz von Kupplungsreagentien herzustellen. Die direkte thermische Kondensation vo.. Alkylphenylcarbonsäuren und sekundären Aminen erfordert nach klassischen Batch-Verfahren jedoch sehr lange Reaktionszeiten von bis zu mehreren Tagen bei Temperaturen von oftmals mehr als 300°C und führt zu keinen befriedigenden Resultaten, da verschiedene Nebenreaktionen die Ausbeute mindern. Dazu zählen beispielsweise eine Decarboxylierung der Carbonsäure, eine Oxidation der Aminogruppe während des langen Erhitzens und insbesondere ein thermisch induzierter Abbau der sekundären Aminogruppe. Die Menge der gebildeten Nebenprodukte erfordert zudem aufwendige Aufarbeitungsschritte. M.S. Nery et al., Synthesis 2003/(2), 272-276 beschriebt die Umwandlung von Carbonsäuren in Amide in Gegenwart von Niobiumpentachlorid.

Ein neuerer Ansatz zur Synthese von Amiden ist die durch Mikrowellen unterstützte Umsetzung von Carbonsäuren und Aminen zu Amiden. So offenbaren Gelens et al:, Tetrahedron Letters 2005, 46(21), 3751-3754, die Synthesen einer Vielzahl von Amiden, die unter Bestrahlung durch Mikrowellen durchgeführt wurden. Die Umsetzungen von Carbonsäuren mit elektronenziehenden Substituenten wie beispielsweise dem Arylrest (Benzoesäure) erfordern dabei bei sehr hohe Reaktionstemperaturen von 250 bis 300°C und führen trotzdem nur zu mäßigen Umsetzungsgraden. Besonders problematisch sind die Umsetzungen von Benzoesäure mit Dialkylaminen, die zu tertiären Amiden führen. So führt die Umsetzung von Benzoesäure mit Di-(n-propyl)amin bei 250°C nur zu 10 % Diamid; sie kann durch Erhöhung der Reaktionstemperatur auf 50 % erhöht werden. Die entsprechende Umsetzung mit Dibenzylamin führt bei 250°C zu einer Ausbeute an Dibenzylamid von nur 25 %; weitere Temperaturerhöhung auf 300°C führt hauptsächlich zu Decarboxylierung der eingesetzten Benzoesäure und nicht zum tertiären Amid. Derartige Umsetzungsgrade sind für technische Prozesse viel zu niedrig. Die Decarboxylierung ist dabei unter kommerziellen wie auch ökologischen Aspekten besonders nachteilig, da die dabei gebildeten aromatischen Kohlenwasserstoffe nicht in den Prozess recyclisiert werden können, sondern entsorgt werden müssen.

Aufgabe vorliegender Erfindung war es, ein Verfahren zur Herstellung von tertiären Amiden von Alkylphenylcarbonsäuren zu finden, bei dem Alkylphenylcarbonsäure und sekundäres Amin direkt und in hohen, das heißt bis zu quantitativen Ausbeuten zum tertiären Amid umgesetzt werden können. Weiterhin sollen dabei keine bzw. nur untergeordnete Mengen an Nebenprodukten wie sekundäre Amide und/oder decarboxylierte Carbonsäuren anfallen.

Überraschenderweise wurde gefunden, dass sich tertiäre Amide von Alkylphenylcarbonsäuren durch direkte Umsetzung von sekundären Aminen mit Alkylphenylcarbonsäuren durch Bestrahlung mit Mikrowellen in hohen Ausbeuten und mit hoher Reinheit herstellen lassen. Überraschenderweise tritt bei Substitution des aromatischen Systems mit mindestens einer Alkylgruppe praktisch keine Decarboxylierung der Arylcarbonsäure auf. Weiterhin findet nur eine geringfügige Eliminierung an der Aminogruppe statt und die Reaktionsprodukte sind annähernd farblos.

Gegenstand der Erfindung ist ein Verfahren zur Herstellung tertiärer Amide von Alkylphenylcarbonsäuren, indem mindestens ein sekundäres Amin mit mindestens einer Alkylphenylcarbonsäure zu einem Ammoniumsalz umgesetzt wird, und dieses Ammoniumsalz nachfolgend unter Mikrowellenbestrahlung weiter zum tertiären Amid umgesetzt wird.

Unter tertiären Amiden werden Amide verstanden, deren Amidstickstoffatom zwei Kohlenwasserstoffreste trägt.

Unter Alkylphenylcarbonsäuren werden solche Säuren verstanden, die mindestens eine Carboxylgruppe und mindestens einen Alkylrest direkt an ein aromatisches System mit (4n + 2) π-Elektronen gebunden enthalten, worin n eine natürliche Zahl und vorzugsweise 1, 2, 3, 4 oder 5 ist. Beispiele für solche aromatischen Systeme sind Benzol, Naphthalin und Phenanthren. Das aromatische System kann neben Carboxyl- und Alkylgruppe ein oder mehrere wie beispielsweise eins, zwei, drei oder mehr gleiche oder verschiedene weitere Substituenten tragen. Geeignete weitere Substituenten sind beispielsweise halogenierte Alkylreste, Hydroxy-, Hydroxyalkyl-, Alkoxy-, Halogen-, Cyano-, Nitril, Nitro- und oder Sulfonsäuregruppen. Diese können an beliebiger Position des aromatischen Systems gebunden sein.

Besonders bevorzugt wird das erfindungsgemäße Verfahren für die Amidierung von aromatischen Carbonsäuren angewendet, die ein aromatisches System mit (4n + 2) π-Elektronen umfassen, welches eine Carboxylgruppe und einen Alkylrest mit 1 bis 20 Kohlenstoffatomen tragen, und worin n für eine ganze Zahl von 1 bis 4 steht.

Besonders vorteilhaft ist das Verfahren bei der Amidierung von Alkylbenzoesäuren, die mindestens einen Alkylrest mit 1 bis 20 C-Atomen und insbesondere 1 bis 12 C-Atomen wie beispielsweise 1 bis 4 C-Atomen tragen. Besonders geeignet ist das erfindungsgemäße Verfahren für die Amidierung von o-Tolylsäure, m-Tolylsäure, p-Tolylsäure, o-Ethylbenzoesäure, m-Ethylbenzoesäure, p-Ethylbenzoesäure, o-Propylbenzoesäure, m-Propylbenzoesäure, p-Propylbenzoesäure und 3,4-Dimethylbenzoesäure.

Erfindungsgemäß geeignete sekundäre Amine besitzen mindestens eine Aminogruppe, die zwei Kohlenwasserstoffreste sowie ein Proton zur Ausbildung der Amidbindung trägt.

Bevorzugte Amine entsprechen der Formel

HNR¹R²

worin R¹ und R² unabhängig voneinander für C₁-C₂₄-Alkyl, C₅-C₁₂-Cycloalkyl oder C₇-C₃₀-Aralkyl stehen.

Bevorzugt stehen R¹ und R² unabhängig voneinander für C₁-C₁₂-Alkyl und speziell für C₁-C₆-Alkyl. Die Alkylreste können linear oder verzweigt sein. Die Reste R¹ und R² können durch Heteroatome wie beispielsweise O und/oder S substituiert sein, und/oder solche Heteroatome enthaltende Substituenten tragen. Bevorzugt enthalten sie jedoch nicht mehr als 1 Heteroatom pro 2 C-Atome. So stehen in einer weiteren bevorzugten Ausführungsform R¹ und/oder R² unabhängig voneinander für Polyoxyalkylenreste der Formel

-(B-O)ₘ-R³,

worin
- B: für einen linearen oder verzweigten C₂-C₄-Alkylenrest, insbesondere für eine Gruppe der Formel -CH₂-CH₂- und/oder -CH(CH₃)-CH₂-,
- m: für eine Zahl von 1 bis 100, bevorzugt 2 bis 20 und
- R³: für Wasserstoff, einen Alkylrest mit 1 bis 20 C-Atomen, einen Cycloalkylrest mit 5 bis 12 Ringatomen, einen Arylrest mit 6 bis 12 Ringatomen, einen Aralkylrest mit 7 bis 30 C-Atomen, einen Heteroarylrest mit 5 bis 12 Ringatomen oder einen Hetero-Aralkylrest mit 6 bis 12 C-Atomen steht.

Als R¹ und/oder R² besonders geeignete araliphatische Reste umfassen Ringsysteme mit mindestens 5 Ringgliedern, die über einen C₁-C₆-Alkylrest an den Stickstoff gebunden sind. Sie können Heteroatome wie S, O und N enthalten. Die aromatischen wie auch die araliphatischen Reste können weitere Substituenten wie beispielsweise Alkylreste, Halogenatome, halogenierte Alkylreste, Nitro-, Cyano-, Nitril-, Hydroxyl- und/oder Hydroxyalkylgruppen tragen.

Als R¹ und/oder R² besonders bevorzugt sind niedere Alkylreste wie beispielsweise Methyl, Ethyl, n-Propyl, iso-Propyl, n-Butyl, iso-Butyl oder tert.-Butyl. Insbesondere stehen R¹ und R² gleichzeitig für Ethyl.

Beispiele für geeignete Amine sind Dimethylamin, Diethylamin, Dipropylamin, Dibutylamin, Methylethylamin, Dioctylamin, Didecylamin, Ditetradecylamin, Dihexadecylamin, Dioctadecylamin sowie deren Mischungen.

Das Verfahren ist insbesondere geeignet zur Herstellung von N,N-Diethyl-m-toluamid.

Im erfindungsgemäßen Verfahren können Alkylphenylcarbonsäure und Amin in beliebigen Verhältnissen miteinander zur Reaktion gebracht werden. Besonders geeignet sind molare Verhältnisse zwischen Alkylphenylcarbonsäure und sekundärem Amin von 10 : 1 bis 1 : 100, bevorzugt von 2 : 1 bis 1 : 2, speziell von 1,0 : 1,2 bis 1,2 : 1,0 und insbesondere equimolar.

In vielen Fällen hat es sich als vorteilhaft erwiesen, mit einem Überschuss an sekundärem Amin, das heißt molaren Verhältnissen von Amin zu Alkylphenylcarbonsäure von mindestens 1,01 : 1,00, insbesondere zwischen 1,05 : 1,00 und 100:1 wie beispielsweise zwischen 1,1 : 1,0 und 10 : 1 zu arbeiten. Dabei wird die Säure praktisch quantitativ zum tertiären Amid umgesetzt. Besonders vorteilhaft ist dieses Verfahren, wenn das eingesetzte sekundäre Amin leicht flüchtig ist. Leicht flüchtig heißt hier, dass das Amin einen Siedepunkt bei Normaldruck von vorzugsweise unterhalb 200°C und insbesondere unterhalb 150°C wie beispielsweise unterhalb 100°C besitzt und sich somit destillativ vom Amid abtrennen lässt.

Die Herstellung der Amide erfolgt durch Umsetzung der Alkylphenylcarbonsäure und des sekundären Amins zum Ammoniumsalz und nachfolgender Bestrahlung des Salzes mit Mikrowellen. Das Ammoniumsalz wird dabei bevorzugt in-situ erzeugt und nicht isoliert. Bevorzugt wird der durch die Mikrowellenbestrahlung bedingte Temperaturanstieg durch Regelung der Mikrowellenintensität und/oder Kühlung des Reaktionsgefäßes auf maximal 330°C begrenzt. Besonders bewährt hat sich die Durchführung der Umsetzung bei Temperaturen zwischen 200 und 300°C wie beispielsweise bei Temperaturen zwischen 220 und 270°C.

Die Dauer der Mikrowellenbestrahlung hängt von verschiedenen Faktoren wie dem Reaktionsvolumen, der Geometrie des Reaktionsraumes und dem gewünschten Umsetzungsgrad ab. Üblicherweise wird die Mikrowellenbestrahlung über einen Zeitraum von weniger als 30 Minuten, bevorzugt zwischen 0,01 Sekunde und 15 Minuten, besonders bevorzugt zwischen 0,1 Sekunde und 10 Minuten und insbesondere zwischen einer Sekunde und 5 Minuten wie beispielsweise zwischen 5 Sekunden und 2 Minuten vorgenommen. Die Intensität (Leistung) der Mikrowellenstrahlung wird dabei so eingestellt, dass das Reaktionsgut in möglichst kurzer Zeit die angestrebte Reaktionstemperatur erreicht. In einer weiteren bevorzugten Ausführungsform des erfindungsgemäßen Verfahrens hat es sich bewährt, das Ammoniumsalz schon vor Beginn der Mikrowellenbestrahlung zu erwärmen, wozu unter anderem die bei der Bildung des Ammoniumsalzes frei werdende Reaktionswärme genutzt werden kann.

Besonders bewährt hat sich dabei ein Aufheizen des Ammoniumsalzes auf Temperaturen zwischen 40 und 200°C, bevorzugt jedoch auf Temperaturen unterhalb des Siedepunkts des Systems. Zum anschließenden Aufrechterhalten der Temperatur kann das Reaktionsgut mit reduzierter und/oder gepulster Leistung weiter bestrahlt werden. Zur Einhaltung der Maximaltemperatur bei gleichzeitig größtmöglicher Mikrowelleneinstrahlung hat es sich bewährt, das Reaktionsgut beispielsweise mittels Kühlmantel, im Reaktionsraum befindliche Kühlrohre durch intermittierende Kühlung zwischen verschiedenen Bestrahlungszonen und/oder durch Siedekühlung über externe Wärmetauscher zu kühlen. In einer bevorzugten Ausführungsform wird das Umsetzungsprodukt direkt nach Beendigung der Mikrowellenbestrahlung möglichst schnell auf Temperaturen unterhalb 120°C, bevorzugt unterhalb 100°C und speziell unterhalb 50°C abgekühlt.

Bevorzugt wird die Umsetzung bei Drücken zwischen 0,1 und 200 bar und speziell zwischen 1 bar (Atmosphärendruck) und 50 bar durchgeführt. Besonders bewährt hat sich das Arbeiten in geschlossenen Gefäßen, in denen oberhalb des Siedepunkts der Edukte bzw. Produkte, des gegebenenfalls anwesenden Lösemittels und/oder oberhalb des während der Reaktion gebildeten Reaktionswassers gearbeitet wird. Üblicherweise reicht der sich auf Grund der Erwärmung des Reaktionsansatzes einstellende Druck zur erfolgreichen Durchführung des erfindungsgemäßen Verfahrens aus. Es kann aber auch unter erhöhtem Druck und/oder unter Anlegen eines Druckprofils gearbeitet werden. In einer weiteren bevorzugten Variante des erfindungsgemäßen Verfahrens wird unter Atmosphärendruck, wie er sich zum Beispiel im offenen Gefäß einstellt, gearbeitet.

Zur Vermeidung von Nebenreaktionen und zur Herstellung von möglichst reinen Produkten hat es sich bewährt, das erfindungsgemäße Verfahren in Gegenwart eines inerten Schutzgases wie beispielsweise Stickstoff, Argon oder Helium durchzuführen.

In einer bevorzugten Ausführungsform wird zur Beschleunigung bzw. zur Vervollständigung der Reaktion in Gegenwart von dehydratisierenden Katalysatoren gearbeitet. Vorzugsweise arbeitet man dabei in Gegenwart eines sauren anorganischen, metallorganischen oder organischen Katalysators oder Gemischen aus mehreren dieser Katalysatoren.

Als saure anorganische Katalysatoren im Sinne der vorliegenden Erfindung sind beispielsweise Borsäure, Schwefelsäure, Phosphorsäure, Polyphosphorsäure, Phosphonsäure, hypophosphorige Säure, Aluminiumsulfathydrat, Alaun, saures Kieselgel, saures Aluminiumhydroxid und Zinkchlorid zu nennen. Besonders bewährt hat sich der Einsatz von Borsäure, Phosphorsäure, Polyphosphorsäure bzw. Zinkchlorid.

Weiterhin und besonders bevorzugt werden Aluminiumverbindungen der allgemeinen Formel Al(OR⁵)₃ und insbesondere Titanate der allgemeinen Formel Ti(OR⁵)₄ als saure anorganische Katalysatoren eingesetzt. Die Reste R⁵ können jeweils gleich oder verschieden sein und unabhängig voneinander gewählt werden aus C₁-C₁₀-Alkylresten, beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, sec.-Hexyl, n-Heptyl, n-Octyl, 2-Ethylhexy, n-Nonyl oder n-Decyl, C₃-C₁₂-Cycloalkylresten, beispielsweise Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl. Bevorzugt sind die Reste R⁵ in Al(OR⁵)₃ bzw. Ti(OR⁵)₄ jeweils gleich und gewählt aus Isopropyl, Butyl und 2-Ethylhexyl.

Bevorzugte saure metallorganische Katalysatoren sind beispielsweise gewählt aus Dialkylzinnoxiden (R⁵)₂SnO, wobei R⁵ wie oben stehend definiert ist. Ein besonders bevorzugter Vertreter für saure metallorganische Katalysatoren ist Di-n-butylzinnoxid, das als so genanntes Oxo-Zinn oder als Fascat^{®}-Marken kommerziell erhältlich ist.

Bevorzugte saure organische Katalysatoren sind saure organische Verbindungen mit beispielsweise Phosphatgruppen, Sulfonsäuregruppen, Sulfatgruppen oder Phosphonsäuregruppen. Besonders bevorzugte Sulfonsäuren enthalten mindestens eine Sulfonsäuregruppe und mindestens einen gesättigten oder ungesättigten, linearen, verzweigten und/oder zyklischen Kohlenwasserstoffrest mit 1 bis 40 C-Atomen und bevorzugt mit 3 bis 24 C-Atomen. Insbesondere bevorzugt sind aromatische Sulfonsäuren, speziell alkylaromatische Mono-Sulfonsäuren mit einem oder mehreren C₁-C₂₈-Alkylresten und insbesondere solche mit C₃-C₂₂-Alkylresten. Geeignete Beispiele sind Methansulfonsäure, Butansulfonsäure, Benzolsulfonsäure, p-Toluolsulfonsäure, Xylolsulfonsäure, 2-Mesitylensulfonsäure, 4-Ethylbenzolsulfonsäure, Isopropylbenzolsulfonsäure, 4-Butylbenzolsulfonsäure, 4-Octylbenzolsulfonsäure; Dodecylbenzolsulfonsäure, Didodecylbenzolsulfonsäure, Naphthalinsulfonsäure. Auch saure Ionenaustauscher können als saure organische Katalysatoren eingesetzt werden, beispielsweise Sulfonsäuregruppen-haltige Poly(styrol)-Harze, die mit etwa 2 mol-% Divinylbenzol vernetzt sind.

Besonders bevorzugt für die Durchführung des erfindungsgemäßen Verfahrens sind Borsäure, Phosphorsäure, Polyphosphorsäure und Polystyrolsulfonsäuren. Insbesondere bevorzugt sind Titanate der allgemeinen Formel Ti(OR⁵)₄ und speziell Titantetrabutylat und Titantetraisopropylat.

Wünscht man saure anorganische, metallorganische oder organische Katalysatoren einzusetzen, so setzt man erfindungsgemäß 0,01 bis 10,0 Gew.-%, bevorzugt 0,05 bis 5,0 Gew.-% wie beispielsweise 0,1 bis 2,0 Gew.-% Katalysator bezogen auf die Masse an eingesetzten Edukten ein. In einer besonders bevorzugten Ausführungsform wird ohne Katalysator gearbeitet.

In einer weiteren bevorzugten Ausführungsform wird die Mikrowellenbestrahlung in Gegenwart von sauren, festen Katalysatoren durchgeführt. Dabei wird der feste Katalysator in dem gegebenenfalls mit Lösemittel versetzten Ammoniumsalz suspendiert oder insbesondere bei kontinuierlichen Verfahren das gegebenenfalls mit Lösemittel versetzte Ammoniumsalz über einen Festbettkatalysator geleitet und der Mikrowellenstrahlung ausgesetzt. Geeignete feste Katalysatoren sind beispielsweise Zeolithe, Kieselgel und Montmorillonit oder auch (teil)vemetzte Polystyrolsulfonsäuren, die gegebenenfalls mit katalytisch aktiven Metallsalzen imprägniert sein können. Geeignete saure Ionentauscher auf Basis vernetzter Polystyrolsulfonsäuren, die als Festphasenkatalysatoren eingesetzt werden können, sind beispielsweise von der Firma Rohm&Haas unter der Markenbezeichnung Amberlyst^{®} erhältlich.

Es hat sich bewährt, in Gegenwart von Lösemitteln zu arbeiten um beispielsweise die Viskosität des Reaktionsmediums abzusenken, das Reaktionsgemisch, sofern es heterogen ist, zu fluidisieren und/oder die Wärmeabfuhr beispielsweise mittels Siedekühlung zu verbessern. Dafür können prinzipiell alle Lösemittel eingesetzt werden, die unter den angewendeten Reaktionsbedingungen inert sind und nicht mit den Edukten bzw. den gebildeten Produkten reagieren. Ein wichtiger Faktor bei der Auswahl geeigneter Lösemittel ist deren Polarität, die einerseits die Löseeigenschaften und andererseits das Ausmaß der Wechselwirkung mit Mikrowellenstrahlung bestimmt. Ein besonders wichtiger Faktor bei der Auswahl geeigneter Lösemittel ist deren dielektrischer Verlust ε". Der dielektrische Verlust ε" beschreibt den Anteil an Mikrowellenstrahlung, der bei der Wechselwirkung einer Substanz mit Mikrowellenstrahlung in Wärme überführt wird. Letzt genannter Wert hat sich als besonders wichtiges Kriterium für die Eignung eines Lösemittels für die Durchführung des erfindungsgemäßen Verfahrens erwiesen. Besonders bewährt hat sich das Arbeiten in Lösemitteln, die eine möglichst geringe Mikrowellenabsorption zeigen und somit nur einen kleinen Beitrag zur Erwärmung des Reaktionssystems liefern. Für das erfindungsgemäße Verfahren bevorzugte Lösemittel besitzen einen bei Raumtemperatur und 2450 MHz gemessenen dielektrischen Verlust ε" von weniger als 10 und vorzugsweise weniger als 1 wie beispielsweise weniger als 0,5. Eine Übersicht über den dielektrischen Verlust verschiedener Lösemittel findet sich zum Beispiel in "Microwave Synthesis" von B. L. Hayes, CEM Publishing 2002. Für das erfindungsgemäße Verfahren geeignet sind insbesondere Lösemittel mit ε"-Werten unterhalb 10 wie N-Methylpyrrolidon, N,N-Dimethylformamid oder Aceton, und insbesondere Lösemittel mit ε"-Werten unterhalb 1. Beispiele für besonders bevorzugte Lösemittel mit ε"-Werten unterhalb 1 sind aromatische und/oder aliphatische Kohlenwasserstoffe wie beispielsweise Toluol, Xylol, Ethylbenzol, Tetralin, Hexan, Cyclohexan, Decan, Pentadecan, Dekalin sowie kommerzielle Kohlenwasserstoffgemische wie Benzinfraktionen, Kerosin, Solvent Naphtha, ^{®}Shellsol AB, ^{®}Solvesso 150, ^{®}Solvesso 200, ^{®}Exxsol, ^{®}Isopar und ^{®}Shellsol-Typen. Lösemittelgemische, die ε"-Werte bevorzugt unterhalb 10 und speziell unterhalb 1 aufweisen, sind für die Durchführung des erfindungsgemäßen Verfahrens gleichermaßen bevorzugt. Prinzipiell ist das erfindungsgemäße Verfahren auch in Lösemitteln mit ε"-Werten von 10 und höher möglich, doch erfordert dies besondere Maßnahmen zur Einhaltung der Maximaltemperatur und führt oftmals zu verminderten Ausbeuten. Sofern in Gegenwart von Lösemitteln gearbeitet wird, liegt deren Anteil an der Reaktionsmischung bevorzugt zwischen 2 und 95 Gew.-%, speziell zwischen 5 und 90 Gew.-% und insbesondere zwischen 10 und 75 Gew.-% wie beispielsweise zwischen 30 und 60 Gew.-%. Besonders bevorzugt wird die Reaktion lösemittelfrei durchgeführt.

Die Mikrowellenbestrahlung wird üblicherweise in Geräten durchgeführt, die einen Reaktionsraum aus einem für Mikrowellen weitestgehend transparenten Material besitzen, in den in einem Mikrowellengenerator erzeugte Mikrowellenstrahlung über geeignete Antennensysteme eingekoppelt wird. Mikrowellengeneratoren, wie beispielsweise das Magnetron und das Klystron sind dem Fachmann bekannt.

Als Mikrowellen werden elektromagnetische Strahlen mit einer Wellenlänge zwischen etwa 1 cm und 1m und Frequenzen zwischen etwa 300 MHz und 30 GHz bezeichnet. Dieser Frequenzbereich ist prinzipiell für das erfindungsgemäße Verfahren geeignet. Bevorzugt wird für das erfindungsgemäße Verfahren Mikrowellenstrahlung mit den für industrielle, wissenschaftliche und medizinische Anwendungen freigegebenen Frequenzen von 915 MHz, 2,45 GHz, 5,8 GHz oder 27,12 GHz verwendet. Es kann sowohl im Mono- bzw. Quasi-Monomode wie auch im Multimode gearbeitet werden. Beim Monomode, der hohe Anforderungen an Geometrie und Größe von Apparatur und Reaktionsraum stellt, wird dabei durch eine stehende Welle insbesondere an deren Maximum eine sehr hohe Energiedichte erzeugt. Beim Multimode dagegen wird der gesamte Reaktionsraum weitgehend homogen bestrahlt, was zum Beispiel größere Reaktionsvolumina ermöglicht.

Die für die Durchführung des erfindungsgemäßen Verfahrens in das Reaktionsgefäß einzustrahlende Mikrowellenleistung ist insbesondere abhängig von der Geometrie des Reaktionsraums und damit des Reaktionsvolumens sowie der Dauer der erforderlichen Bestrahlung. Sie liegt üblicherweise zwischen 100 W und mehreren 100 kW und insbesondere zwischen 200 W und 100 kW wie beispielsweise zwischen 500 W und 70 kW. Sie kann an einer oder mehreren Stellen des Reaktors appliziert werden. Sie kann über einen oder mehrere Mikrowellengeneratoren erzeugt werden.

Die Reaktion kann diskontinuierlich im Batch-Verfahren oder, bevorzugt, kontinuierlich zum Beispiel in einem Strömungsrohr durchgeführt werden. Sie kann weiterhin in semi-Batch Prozessen wie beispielsweise kontinuierlich betriebenen Rührreaktoren oder Kaskadenreaktoren durchgeführt werden. In einer bevorzugten Ausführungsform wird die Reaktion in einem geschlossenen Gefäß durchgeführt, wobei das sich bildende Kondensat sowie gegebenenfalls Edukte und, sofern anwesend, Lösemittel zu einem Druckaufbau führen. Nach Beendigung der Reaktion kann der Überdruck durch Entspannung zur Verflüchtigung und Abtrennung von Reaktionswasser und gegebenenfalls Lösemittel sowie überschüssigen Edukten und/oder Abkühlung des Reaktionsprodukts verwendet werden. In einer weiteren Ausführungsform wird das gebildete Reaktionswasser nach dem Abkühlen und/oder Entspannen durch übliche Verfahren wie beispielsweise Phasentrennung, Destillation und/oder Absorption abgetrennt. Das erfindungsgemäße Verfahren kann ebenso erfolgreich in einem offenen Gefäß unter Siedekühlung und/oder Auskreisen des Reaktionswassers erfolgen.

In einer bevorzugten Ausführungsform wird das erfindungsgemäße Verfahren in einem diskontinuierlichen Mikrowellenreaktor durchgeführt. Dabei wird die Mikrowellenbestrahlung in einem gerührten Gefäß vorgenommen. Bevorzugt befinden sich zur Abführung überschüssiger Wärme im Reaktionsgefäß Kühlelemente wie beispielsweise Kühlfinger oder Kühlschlangen oder an das Reaktionsgefäß angeflanscht Rückflusskühler zur Siedekühlung des Reaktionsmediums. Für die Bestrahlung größerer Reaktionsvolumina wird die Mikrowelle hier bevorzugt im Multimode betrieben. Die diskontinuierliche Ausführungsform des erfindungsgemäßen Verfahrens erlaubt durch Variation der Mikrowellenleistung schnelle wie auch langsame Heizraten und insbesondere das Halten der Temperatur über längere Zeiträume wie beispielsweise mehrere Stunden. Die Reaktanden und gegebenenfalls Lösemittel und weitere Hüfsstoffe können vor Beginn der Mikrowellenbestrahlung im Reaktionsgefäß vorgelegt werden. Bevorzugt haben sie dabei Temperaturen unterhalb 100°C wie beispielsweise zwischen 10 und 50°C. In einer bevorzugten Ausführungsform werden die Reaktanden oder Teile davon dem Reaktionsgefäß erst während der Bestrahlung mit Mikrowellen zugeführt. In einer weiteren bevorzugten Ausführungsform wird der diskontinuierliche Mikrowellenreaktor unter kontinuierlichem Zuführen von Edukten und gleichzeitigem Ausschleusen von Reaktionsgut in Form eines Semi-Batch- bzw. Kaskadenreaktors betrieben.

In einer besonders bevorzugten Ausführungsform wird das erfindungsgemäße Verfahren in einem kontinuierlichen Mikrowellenreaktor durchgeführt. Die Reaktionsmischung wird dazu durch ein druckfestes, gegenüber den Reaktanden inertes, für Mikrowellen weitestgehend transparentes und in einen Mikrowellenofen eingebautes Reaktionsrohr geführt. Dieses Reaktionsrohr hat bevorzugt einen Durchmesser von einem Millimeter bis ca. 50 cm, speziell zwischen 2 mm und 35 cm wie beispielsweise zwischen 5 mm und 15 cm. Unter Reaktionsrohren werden hier Gefäße verstanden, deren Verhältnis von Länge zu Durchmesser größer als 5, bevorzugt zwischen 10 und 100:000, besonders bevorzugt zwischen 20 und 10.000 wie beispielsweise zwischen 30 und 1.000 ist. In einer speziellen Ausführungsform ist das Reaktionsrohr in Form eines Doppelmantelrohres ausgestaltet, durch dessen Innen- und Außenraum die Reaktionsmischung nacheinander im Gegenstrom geführt werden kann, um beispielsweise die Temperaturführung und Energieeffizienz des Verfahrens zu erhöhen. Als Länge des Reaktionsrohres ist dabei die vom Reaktionsgemisch insgesamt durchströmte Strecke zu verstehen. Das Reaktionsrohr ist auf seiner Länge von mindestens einem, bevorzugt aber von mehreren wie beispielsweise zwei, drei, vier, fünf, sechs, sieben acht oder mehr Mikrowellenstrahlern umgeben. Die Mikrowelleneinstrahlung erfolgt bevorzugt über den Rohrmantel. In einer weiteren bevorzugten Ausführungsform erfolgt die Mikrowelleneinstrahlung mittels mindestens einer Antenne über die Rohrenden. Das Reaktionsrohr ist üblicherweise am Einlass mit einer Dosierpumpe sowie einem Manometer und am Auslass mit einem Druckhalteventil und einem Wärmetauscher versehen. Bevorzugt werden die Edukte Amin und Alkylphenylcarbonsäure, beide unabhängig voneinander gegebenenfalls mit Lösemittel verdünnt, erst kurz vor dem Eintritt in das Reaktionsrohr vermischt. Weiterhin bevorzugt werden die Edukte dem erfindungsgemäßen Verfahren in flüssiger Form mit Temperaturen unterhalb 100°C wie beispielsweise zwischen 10°C und 50°C zugeführt. Dazu können höher schmelzende Edukte beispielsweise in geschmolzenem Zustand oder mit Lösemittel versetzt eingesetzt werden.

Durch Variation von Rohrquerschnitt, Länge der Bestrahlungszone (hierunter wird der Anteil des Reaktionsrohres verstanden, in dem das Reaktionsgut Mikrowellenstrahlung ausgesetzt ist), Fließgeschwindigkeit, Geometrie der Mikrowellenstrahler, der eingestrahlten Mikrowellenleistung sowie der dabei erreichten Temperatur werden die Reaktionsbedingungen so eingestellt, dass die maximale Reaktionstemperatur schnellstmöglich erreicht wird und die Verweilzeit bei Maximaltemperatur so kurz bleibt, dass so wenig Neben- oder Folgereaktionen wie möglich auftreten. Bevorzugt wird der kontinuierliche Mikrowellenreaktor im Monomode oder Quasi-Monomode betrieben. Die Verweilzeit im Reaktionsrohr liegt dabei im Allgemeinen unter 30 Minuten, bevorzugt zwischen 0,01 Sekunden und 15 Minuten, bevorzugt zwischen 0,1 Sekunden und 5 Minuten wie beispielsweise zwischen einer Sekunde und 3 Minuten. Das Reaktionsgut kann zur Vervollständigung der Reaktion, gegebenenfalls nach Zwischenkühlung, mehrfach den Reaktor durchlaufen. Es hat sich besonders bewährt, wenn das Reaktionsprodukt unmittelbar nach Verlassen des Reaktionsrohres z. B. durch Mantelkühlung oder Entspannung abgekühlt wird.

Dabei war es besonders überraschend, dass trotz der im kontinuierlich durchflossenen Strömungsrohr nur sehr kurzen Verweildauer des Ammoniumsalzes im Mikrowellenfeld eine so weitgehende Amidierung ohne Bildung nennenswerter Mengen an Nebenprodukten stattfindet. Bei einer entsprechenden Umsetzung dieser Ammoniumsalze in einem Strömungsrohr unter thermischer Mantelheizung werden zur Erzielung geeigneter Reaktionstemperaturen extrem hohe Wandtemperaturen benötigt, die zur Bildung gefärbter Spezies führten, aber praktisch keine Amidbildung bewirken.

Zur Vervollständigung der Umsetzung hat es sich in vielen Fällen bewährt, das erhaltene Rohprodukt vom Reaktionswasser zu trocknen und erneut der Mikrowellenbestrahlung auszusetzen. In einer weiteren bevorzugten Ausführungsform hat es sich bewährt, unumgesetzte Edukte nach Abtrennung vom Reaktionsprodukt in das erfindungsgemäße Verfahren zurückzuführen, was zu einer praktisch quantitativen Umsetzung der eingesetzten Edukte und insbesondere der Alkylphenylcarbonsäure führt.

Üblicherweise fallen über den erfindungsgemäßen Weg hergestellte tertiäre Amide in einer für die weitere Verwendung ausreichenden Reinheit an. Für spezielle Anforderungen können sie jedoch nach üblichen Reinigungsverfahren wie Destillation, Umkristallisation, Filtration bzw. chromatographische Verfahren weiter aufgereinigt werden.

Das erfindungsgemäße Verfahren erlaubt eine sehr schnelle und kostengünstige Herstellung tertiärer Amide von Alkylphenylcarbonsäuren in hohen Ausbeuten und mit hoher Reinheit. Dabei fallen keine wesentlichen Mengen an Nebenprodukten an. Die nach dem erfindungsgemäßen Verfahren hergestellten Produkte sind zudem annähernd farblos, das heißt sie besitzen lod-Farbzahlen von unter 5 und oftmals unter 2 wie beispielsweise zwischen 0,1 und 1,5. Durch thermische Kondensation im Autoklaven hergestellte Produkte haben dagegen üblicherweise lod-Farbzahlen oberhalb 30 bzw. sind oftmals gar nicht mehr messbar. Daher sind für nach dem erfindungsgemäßen Verfahren hergestellte Produkte üblicherweise keine Auf- oder Nacharbeitungsschritte erforderlich. Besonders überraschend war die Beobachtung, dass Alkylphenylcarbonsäuren unter den Bedingungen des erfindungsgemäßen Verfahrens keine merkliche Decarboxylierung zeigen. Derartig schnelle und selektive Umsetzungen sind nach klassischen Methoden nicht zu erzielen und waren alleine durch Heizen auf hohe Temperaturen auch nicht zu erwarten. Die erfindungsgemäß hergestellten tertiären Amide von Alkylphenylcarbonsäuren eignen sich insbesondere als Insektenrepellents. Da die nach dem erfindungsgemäßen Verfahren hergestellten tertiären Amide verfahrensbedingt keine Reste von Kupplungsreagentien bzw. deren Folgeprodukten enthalten, können sie problemlos auch in toxikologisch sensiblen Bereichen wie beispielsweise kosmetischen und pharmazeutischen Zubereitungen eingesetzt werden.

### Beispiele

Die Umsetzungen unter Mikrowellenbestrahlung erfolgten in einem Single-Mode Mikrowellenreaktor vom Typ "Discover" der Firma CEM bei einer Frequenz von 2,45 GHz. Kühlung der Reaktionsgefäße erfolgte mittels Druckluft. Die Temperaturmessung musste auf Grund der Druckbedingungen in den Reaktionsgefäßen über einen IR-Sensor am Küvettenboden erfolgen. Durch Vergleichsversuche mit einer in die Reaktionsmischung eintauchenden Glasfiberoptik wurde festgestellt, dass die Temperatur im Reaktionsmedium im hier relevanten Temperaturbereich etwa 50 bis 80°C über der mit dem IR-Sensor am Küvettenboden gemessenen Temperatur liegt.

Die diskontinuierlich durchgeführten Umsetzungen erfolgten in geschlossenen, druckfesten Glasküvetten mit einem Volumen von 8 ml unter Magnetrührung: Kontinuierlich durchgeführte Umsetzungen erfolgten in druckfesten, zylindrischen, als Doppelmantelrohr ausgestalteten Glasküvetten (ca. 10 x 1,5 cm; Reaktionsvolumen ca. 15 ml) mit innen liegendem, über dem Küvettenboden endendem Einleitungsrohr und Produktabnahme am oberen Ende der Küvette. Der sich während der Reaktion aufbauende Druck wurde über ein Druckhalteventil auf maximal 20 bar begrenzt und in eine Vorlage entspannt. Das Ammoniumsalz wurde durch das Einleitungsrohr in die Küvette gepumpt und die Verweilzeit in der Bestrahlungszone durch Modifizierung der Pumpenleistung auf etwa 1 Minute eingestellt.

Die Analytik der Produkte erfolgte mittels ¹H-NMR-Spektroskopie bei 500 MHz in Pyridin-d₅ bzw. mittels GC/MS. Die Nachweisgrenze für aromatische Kohlenwasserstoffe lag dabei bei ca. 1 %. Wasserbestimmungen erfolgten mittels Karl-Fischer-Titration.

### Beispiel 1: Herstellung von N,N-Diethyl-m-toluamid

1 g Diethylamin wurde unter Kühlung langsam mit einer äquimolaren Menge (1,9 g) m-Tolylsäure versetzt und gemischt. Nach Abklingen der Wärmetönung wurde das so erhaltene Ammoniumsalz in einer geschlossenen Küvette für 5 Minuten unter maximaler Kühlleistung einer Mikrowellenbestrahlung von 150 W ausgesetzt. Es wurde eine mittels IR-Sensor gemessene Temperatur von 160°C erreicht; der Druck stieg auf etwa 14 bar. Anschließend wurde die Reaktionsmischung binnen 2 Minuten auf 30°C abgekühlt.

Das erhaltene Rohprodukt enthielt als Hauptkomponenten 66 % N,N-Diethyl-m-toluamid, 2 % N-Ethyl-m-toluamid, 6 % Wasser sowie unumgesetzte Edukte. Nach Trocknung des Reaktionsansatzes über Molekularsieb, erneute einminütige Bestrahlung mit Mikrowellen von 150 W und Trocknung über Molekularsieb wurde ein 92 %iger Umsatz der m-Tolylsäure zu N,N-Diethyl-m-toluamid erzielt. Es konnte kein Toluol als thermisches Spaltprodukt nachgewiesen werden. Die lod-Farbzahl lag bei 3.

### Beispiel 2: Herstellung von N,N-Diethyl-m-toluamid unter Katalyse durch Borsäure/p-Toluolsulfonsäure

0,53 g Diethylamin wurden unter Kühlung langsam mit einer äquimolaren Menge (1,0 g) m-Tolylsäure versetzt und gemischt. Nach Abklingen der Wärmetönung wurde das so erhaltene Ammoniumsalz mit 15,6 mg Borsäure und 15 mg p-Toluolsulfonsäure versetzt und in einer geschlossenen Küvette für 5 Minuten unter maximaler Kühlleistung einer Mikrowellenbestrahlung von 75 W ausgesetzt. Es wurde eine mittels IR-Sensor gemessene Temperatur von 200°C erreicht; der Druck stieg auf 20 bar. Anschließend wurde die Reaktionsmischung binnen 2 Minuten auf 30°C abgekühlt.

Das erhaltene Rohprodukt enthielt als Hauptkomponenten 75 % N,N-Diethyl-m-toluamid und 8 % N-Ethyl-m-toluamid und unumgesetzte Edukte sowie 6,5 % Wasser. Es konnte kein Toluol als thermisches Spaltprodukt nachgewiesen werden. Die lod-Farbzahl lag bei 4.

### Beispiel 3: Herstellung von N,N-Diethyl-m-toluamid unter Katalyse durch Titantetrabutylat

2 g Diethylamin wurden unter Kühlung langsam mit 1 g m-Tolylsäure versetzt und gemischt. Nach Abklingen der Wärmetönung wurde das so erhaltene Ammoniumsalz mit 30 mg Titantetrabutylat versetzt und in einer geschlossenen Küvette für 2 Minuten unter maximaler Kühlleistung einer Mikrowellenbestrahlung von 150 W ausgesetzt. Es wurde eine mittels IR-Sensor gemessene Temperatur von 200°C erreicht; der Druck stieg auf 20 bar. Anschließend wurde die Reaktionsmischung binnen 2 Minuten auf 30°C abgekühlt.

Im erhaltenen Rohprodukt waren 81 % der Tolylsäure zu N,N-Diethyl-m-toluamid und weiter 9 % zu N-Ethyl-m-toluamid umgesetzt. Nach Entfernen des Reaktionswassers und erneuter Bestrahlung sowie anschließender destillativer Abtrennung von Wasser und überschüssigem Diethylamin wurde ein 90 %iges N,N-Diethyl-m-toluamid erhalten. Es konnte kein Toluol als thermisches Spaltprodukt nachgewiesen werden. Die lod-Farbzahl des erhaltenen Produkts lag bei 4.

### Beispiel 4: Herstellung von N,N-Dihexyl-m-toluamid mit überschüssigem Dihexylamin

2,5 g Dihexylamin wurden unter Kühlen und Rühren langsam mit 1 g m-Tolylsäure versetzt. Nach Abklingen der Wärmetönung wurde die so erhaltene Lösung des Ammoniumsalzes in einer geschlossenen Küvette für 7 Minuten unter maximaler Kühlleistung einer Mikrowellenbestrahlung von 100 W ausgesetzt. Es wurde eine mittels IR-Sensor gemessene Temperatur von 190°C bei einem Druck von 16 bar erreicht. Anschließend wurde die Reaktionsmischung binnen 2 Minuten auf 30°C abgekühlt.

Im so erhaltenen Rohprodukt waren 50 % der Tolylsäure zu N,N-Dihexyl-m-toluamid umgesetzt. Nach Trocknung über Molekularsieb wurde es erneut für 5 Minuten mit Mikrowellen bestrahlt. Nach Abdestillieren von überschüssigem Dihexylamin und Reaktionswasser wurden 77 % N,N-Dihexyl-m-toluamid (bezogen auf die eingesetzte m-Tolylsäure) erhalten. Es konnte kein Toluol als thermisches Spaltprodukt nachgewiesen werden. Die lod-Farbzahl des erhaltenen Produkts lag bei 3.

### Beispiel 5: Kontinuierliche Herstellung von N,N-Diethyl-m-toluamid

Unter Kühlung und Rühren wurden 100 g Diethylamin langsam mit 136 g m-Tolylsäure versetzt. Nach Abklingen der Wärmetönung wurde das so erhaltene Ammoniumsalz kontinuierlich über den Bodeneinlass durch die in der Mikrowellencavität montierte Glasküvette gepumpt. Die Förderleistung der Pumpe wurde dabei so eingestellt, dass die Verweilzeit in der Küvette und damit in der Bestrahlungszone etwa 10 Sekunden betrug. Es wurde unter maximaler Kühlleistung mit einer Mikrowellenleistung von 300 W gearbeitet, wobei eine mittels IR-Sensor gemessene Temperatur von 150°C erreicht wurde. Nach Verlassen der Glasküvette wurde die Reaktionsmischung über einen kurzen Liebig-Kühler auf 30°C abgekühlt.

Das Rohprodukt enthielt eine Ausbeute von 56 % N,N-Diethyl-m-toluamid bezogen auf die eingesetzte m-Tolylsäure. Nach Abtrennen des Reaktionswassers, erneutem Durchlaufen des obigen Prozesses und Abdestillieren von überschüssigem Diethylamin und Reaktionswasser wurde ein Umsatz von 79 % N,N-Diethyl-m-toluamid bezogen auf die eingesetzte m-Tolylsäure erhalten. Es konnte kein Toluol als thermisches Spaltprodukt nachgewiesen werden. Die lod-Farbzahl des erhaltenen Produkts lag bei 1.

### Beispiel 6: Kontinuierliche Herstellung von N,N-Diethyl-m-toluamid

Unter Kühlung und Rühren wurden 73 g Diethylamin (1mol) langsam mit 136 g m-Tolylsäure (1mol) versetzt. Nach Abklingen der Wärmetönung wurde das so erhaltene Ammoniumsalz kontinuierlich über den Bodeneinlass durch die in der Mikrowellencavität montierte Glasküvette gepumpt. Die Förderleistung der Pumpe wurde dabei so eingestellt, dass die Verweilzeit in der Küvette und damit in der Bestrahlungszone etwa 100 Sekunden betrug. Es wurde unter maximaler Kühlleistung mit einer Mikrowellenleistung von 500 W gearbeitet, wobei eine mittels IR-Sensor gemessene Temperatur von 200 °C erreicht wurde. Nach Verlassen der Glasküvette wurde die Reaktionsmischung über einen kurzen Liebig-Kühler auf RT abgekühlt.

Das Rohprodukt enthielt eine Ausbeute von 75 % N,N-Diethyl-m-toluamid bezogen auf die eingesetzte m-Tolylsäure. Es konnte kein Toluol als thermisches Spaltprodukt nachgewiesen werden. Nach Abtrennen des Reaktionswassers und erneutem Durchlaufen des obigen Prozesses wurde ein Umsatz von 88 % N,N-Diethyl-m-toluamid bezogen auf die eingesetzte m-Tolylsäure erhalten. Die lod-Farbzahl des erhaltenen Produkts lag bei 1.

### Beispiel 7: Herstellung von N,N-Diethyl-benzamid (Vergleich 1)

2 g Diethylamin wurden unter Kühlung langsam mit 1 g Benzoesäure versetzt und gemischt. Nach Abklingen der Wärmetönung wurde das so erhaltene Ammoniumsalz in einer geschlossenen Küvette für 5 Minuten unter maximaler Kühlleistung einer Mikrowellenbestrahlung von 200 W ausgesetzt. Es wurde eine mittels IR-Sensor gemessene Temperatur von 230°C erreicht; der Druck stieg auf 20 bar. Anschließend wurde die Reaktionsmischung binnen 2 Minuten auf 30°C abgekühlt.

Im Rohprodukt waren 42 % der eingesetzten Benzoesäure zu N,N-Diethylbenzamid und weitere 15 % zu N-Ethyl-benzamid umgesetzt. Weiterhin fanden sich im Rohprodukt 11 % Benzol, die aus der thermischen Decarboxylierung der eingesetzten Benzoesäure stammen.

### Beispiel 8: Kontinuierliche thermische Umsetzung von m-Tolylsäure und Diethylamin (Vergleich 2)

Unter Kühlung und Rühren wurden 73 g Diethylamin (1mol) langsam mit 136 g m-Tolylsäure (1 mol) versetzt. Nach Abklingen der Wärmetönung wurde das so erhaltene Ammoniumsalz kontinuierlich über den Bodeneinlass durch die sich in einem 300°C heißen Ölbad befindliche druckfeste Glasküvette gepumpt. Die Förderleistung der Pumpe wurde dabei so eingestellt, dass die Verweilzeit der Reaktanden in der Küvette und damit in der Reaktionszone etwa 85 Sekunden betrug. Eine Temperaturmessung wurde am Überlauf der Küvette vorgenommen. Die hierbei beobachteten Maximaltemperaturen betrugen 220°C. Nach Verlassen der Glasküvette wurde die Reaktionsmischung über einen kurzen Liebig-Kühler auf RT abgekühlt.

Die so erhaltene Reaktionsmischung enthielt weniger als 2 mol-% N,N-Diethyl-m-toluamid. Die lod-Farbzahl lag bei 35.

## Patentansprüche

1. Verfahren zur Herstellung tertiärer Amide von Alkylphenylcarbonsäuren, indem mindestens ein sekundäres Amin mit mindestens einer Alkylphenylcarbonsäure zu einem Ammoniumsalz umgesetzt wird, und dieses Ammoniumsalz nachfolgend unter Mikrowellenbestrahlung weiter zum tertiären Amid umgesetzt wird.

2. Verfahren nach Anspruch 1, worin die Alkylphenylcarbonsäure mindestens einen C₁- bis C₂₀-Alkylrest trägt.

3. Verfahren nach Anspruch 1 und/oder 2, worin die Alkylphenylcarbonsäure aus o-Tolylsäure, m-Tolylsäure, p-Tolylsäure, o-Ethylbenzoesäure, m-Ethylbenzoesäure, p-Ethylbenzoesäure, o-Propylbenzoesäure, m-Propylbenzoesäure, p-Propylbenzoesäure und 3,4-Dimethylbenzoesäure ausgewählt ist.

4. Verfahren nach einem oder mehreren der Ansprüche 1 bis 3, worin das Amin der Formel
HNR¹R²
entspricht, worin R¹ und R² unabhängig voneinander für C₁-C₂₄-Alkyl, C₅₋C₁₂-Cycloafkyl oder C₇-C₃₀-Aralkyl stehen, oder für Polyoxyalkylenreste der Formel
-(B-O)ₘ-R³,
stehen, worin
B für einen linearen oder verzweigten C₂-C₄-Alkylenrest, insbesondere für eine Gruppe der Formel -CH₂-CH₂- und/oder-CH(CH₃)-CH₂-,
m für eine Zahl von 1 bis 100, bevorzugt 2 bis 20 und
R³ für Wasserstoff, einen Alkylrest mit 1 bis 20 C-Atomen, einen Cycloalkylrest mit 5 bis 12 Ringatomen, einen Arylrest mit 6 bis 12 Ringatomen, einen Aralkylrest mit 7 bis 30 C-Atomen, einen Heteroarylrest mit 5 bis 12 Ringatomen oder einen Hetero-Aralkylrest mit 6 bis 12 C-Atomen steht.

5. Verfahren nach einem oder mehreren der Ansprüche 1 bis 4, wobei die Mikrowellenbestrahlung in Gegenwart eines dehydratisierenden Katalysators durchgeführt wird.

6. Verfahren nach einem oder mehreren der Ansprüche 1 bis 5, welches in Gegenwart eines Lösemittels durchgeführt wird.

7. Verfahren nach Anspruch 6, wobei das Lösemittel einen ε"-Wert von unter 10 aufweist.

8. Verfahren nach einem oder mehreren der Ansprüche 1 bis 7, wobei die Reaktionstemperatur unterhalb 330°C liegt.

9. Verfahren nach einem oder mehreren der Ansprüche 1 bis 8, wobei die Reaktion bei Drücken zwischen 0,1 und 200 bar durchgeführt wird.

10. Verfahren nach einem oder mehreren der Ansprüche 1 bis 9, wobei die Umsetzung kontinuierlich durch Bestrahlung mit Mikrowellen in einem vom Ammoniumsalz durchströmten Reaktionsrohr erfolgt.

11. Verfahren nach Anspruch 10, wobei das Reaktionsrohr aus einem nicht metallischen; mikrowellentransparenten Werkstoff besteht.

12. Verfahren nach Anspruch 10 und/oder 11, worin die Verweilzeit des Reaktionsgutes im Reaktionsrohr weniger als 30 Minuten beträgt.

13. Verfahren nach einem oder mehreren der Ansprüche 10 bis 12, wobei das Reaktionsrohr ein Verhältnis von Länge zu Durchmesser von mindestens 5 besitzt.

## Claims

1. A process for preparing tertiary amides of alkylphenylcarboxylic acids by reacting at least one secondary amine with at least one alkylphenylcarboxylic acid to give an ammonium salt, and then converting this ammonium salt further under microwave irradiation to the tertiary amide.

2. The process as claimed in claim 1, in which the alkylphenylcarboxylic acid bears at least one C₁- to C₂₀-alkyl radical.

3. The process as claimed in claim 1 and/or 2, in which the alkylphenylcarboxylic acid is selected from o-toluic acid, m-toluic acid, p-toluic acid, o-ethylbenzoic acid, m-ethylbenzoic acid, p-ethylbenzoic acid, o-propylbenzoic acid, m-propylbenzoic acid, p-propylbenzoic acid and 3,4-dimethylbenzoic acid.

4. The process as claimed in one or more of claims 1 to 3, in which the amine is of the formula
HNR¹R²
in which R¹ and R² are each independently C₁-C₂₄-alkyl, C₅-C₁₂-cycloalkyl or C₇-C₃₀-aralkyl, or are each polyoxyalkylene radicals of the formula
-(B-O)ₘ-R³
in which
B is a linear or branched C₂-C₄-alkylene radical, especially a group of the formula -CH₂-CH₂- and/or -CH(CH₃)-CH₂-,
m is from 1 to 100, preferably 2 to 20, and
R³ is hydrogen, an alkyl radical having 1 to 20 carbon atoms, a cycloalkyl radical having 5 to 12 ring atoms, an aryl radical having 6 to 12 ring atoms, an aralkyl radical having 7 to 30 carbon atoms, a heteroaryl radical having 5 to 12 ring atoms or a heteroaralkyl radical having 6 to 12 carbon atoms.

5. The process as claimed in one or more of claims 1 to 4, wherein the microwave irradiation is performed in the presence of a dehydrating catalyst.

6. The process as claimed in one or more of claims 1 to 5, which is performed in the presence of a solvent.

7. The process as claimed in claim 6, wherein the solvent has an ε" value of less than 10.

8. The process as claimed in one or more of claims 1 to 7, wherein the reaction temperature is below 330°C.

9. The process as claimed in one or more of claims 1 to 8, wherein the reaction is performed at pressures between 0.1 and 200 bar.

10. The process as claimed in one or more of claims 1 to 9, wherein the reaction is effected continuously by irradiating with microwaves in a reaction tube through which the ammonium salt flows.

11. The process as claimed in claim 10, wherein the reaction tube consists of a nonmetallic microwave-transparent material.

12. The process as claimed in claim 10 and/or 11, in which the residence time of the reaction mixture in the reaction tube is less than 30 minutes.

13. The process as claimed in one or more of claims 10 to 12, wherein the reaction tube has a ratio of length to diameter of at least 5.

## Revendications

1. Procédé pour la production d'amides tertiaires d'acides alkylphénylcarboxyliques, par mise en réaction d'au moins une amine secondaire avec au moins un acide alkylphénylcarboxylique conduisant à un sel d'ammonium, et ensuite conversion de ce sel d'ammonium à son tour en l'amide tertiaire, sous irradiation avec des micro-ondes.

2. Procédé selon la revendication 1, dans lequel l'acide alkylphénylcarboxylique porte au moins un radical alkyle en C₁-C₂₀.

3. Procédé selon la revendication 1 et/ou la revendication 2, dans lequel l'acide alkylphénylcarboxylique est choisi parmi l'acide o-tolylique, l'acide m-tolylique, l'acide p-tolylique, l'acide o-éthylbenzoïque, l'acide m-éthylbenzoïque, l'acide p-éthylbenzoïque, l'acide o-propylbenzoïque, l'acide m-propylbenzoïque, l'acide p-propylbenzoïque et l'acide 3,4-diméthylbenzoïque.

4. Procédé selon une ou plusieurs des revendications 1 à 3, dans lequel l'amine correspond à la formule
HNR¹R²
dans laquelle R¹ et R² représentent, indépendamment l'un de l'autre, un groupe alkyle en C₁-C₂₄, cycloalkyle en C₅-C₁₂ ou aralkyle en C₇-C₃₀, ou des radicaux polyoxyalkylène de formule
-(B-O)ₘ-R ³
dans laquelle
B représente un radical alkylène en C₂-C₄ linéaire ou ramifié, en particulier un groupe de formule -CH₂-CH₂- et/ou -CH(CH₃)-CH₂-,
m représente un nombre de 1 à 100, de préférence de 2 à 20 et
R³ représente un atome d'hydrogène, un radical alkyle ayant de 1 à 20 atomes de carbone, un radical cycloalkyle ayant de 5 à 12 atomes formant le cycle, un radical aryle ayant de 6 à 12 atomes formant le cycle, un radical aralkyle ayant de 7 à 30 atomes de carbone, un radical hétéroaryle ayant de 5 à 12 atomes formant le cycle ou un radical hétéroaralkyle ayant de 6 à 12 atomes de carbone.

5. Procédé selon une ou plusieurs des revendications 1 à 4, dans lequel l'irradiation avec des micro-ondes est effectuée en présence d'un catalyseur déshydratant.

6. Procédé selon une ou plusieurs des revendications 1 à 5, qui est effectué en présence d'un solvant.

7. Procédé selon la revendication 6, dans lequel le solvant présente une valeur ε" inférieure à 10.

8. Procédé selon une ou plusieurs des revendications 1 à 7, dans lequel la température de réaction est inférieure à 330 °C.

9. Procédé selon une ou plusieurs des revendications 1 à 8, dans lequel la réaction est effectuée sous des pressions comprises entre 0,1 et 200 bars.

10. Procédé selon une ou plusieurs des revendications 1 à 9, dans lequel la réaction s'effectue en continu par irradiation avec des micro-ondes dans un tube de réaction dans lequel passe le sel d'ammonium.

11. Procédé selon la revendication 10, dans lequel le tube de réaction est constitué d'un matériau non métallique, transparent aux micro-ondes.

12. Procédé selon la revendication 10 et/ou la revendication 11, dans lequel le temps de séjour de la matière en réaction dans le tube de réaction est inférieur à 30 minutes.

13. Procédé selon une ou plusieurs des revendications 10 à 12, dans lequel le tube de réaction présente un rapport de la longueur au diamètre d'au moins 5.
